## Europäisches Patentamt

## European Patent Office

## Office européen des brevets

(11) Veröffentlichungsnummer: **0 070 535**
**B1**

(12) **EUROPÄISCHE PATENTSCHRIFT**

(45) Veröffentlichungstag der Patentschrift:
**03.07.85**

(21) Anmeldenummer: **82106407.8**

(22) Anmeldetag: **16.07.82**

(51) Int. Cl.⁴: **C 07 C 67/56, C 07 C 69/30, C 07 C 69/33, C 07 C 69/58**

(54) Verfahren zum Reinigen von Estern.

(30) Priorität: **21.07.81 DE 3128696**

(43) Veröffentlichungstag der Anmeldung:
**26.01.83 Patentblatt 83/4**

(45) Bekanntmachung des Hinweises auf die Patenterteilung:
**03.07.85 Patentblatt 85/27**

(84) Benannte Vertragsstaaten:
**BE CH DE FR GB IT LI NL SE**

(56) Entgegenhaltungen:
**DE - A - 2 326 225**
**GB - A - 705 599**

(73) Patentinhaber: **HOECHST AKTIENGESELLSCHAFT,**
**Postfach 80 03 20, D-6230 Frankfurt am Main 80 (DE)**

(72) Erfinder: **Stühler, Herbert, Dr., Hochfeinstrasse 12,**
**D-8261 Burgkirchen/Alz (DE)**
Erfinder: **Krempl, Engelbert, Hochgernweg 2,**
**D-8261 Burgkirchen/Alz (DE)**

ACTORUM AG

# Beschreibung

Die Erfindung betrifft ein Verfahren zum Reinigen von alkoholhaltigen Estern, mit dem die im Ester enthaltenen Alkohole in einfacher Weise entfernt werden können.

Ester, bei deren Herstellung Alkohole eingesetzt oder gebildet werden, enthalten auch nach den üblichen Aufarbeitungsverfahren zur Abtrennung des Esters aus dem Reaktionsprodukt im allgemeinen noch Restmengen an Alkohol. Selbst bei jenen Herstellungsverfahren, bei denen der Alkohol nicht im Überschuss, sondern im stöchiometrischen Verhältnis eingesetzt wird, und selbst bei jenen Reaktionen, mit denen lediglich eine Modifizierung eines Esters, beispielsweise eine Oxalkylierung angestrebt wird, erhält man Ester, die noch kleine Mengen an Alkohol enthalten.

Die Anwesenheit von Alkohol führt häufig zu einer Trübung der an sich klaren Ester. In diesem Falle liegt der Alkohol im wesentlichen in Form von dispergierten Teilchen (Flüssigkeitströpfchen) im Ester vor. Diese Trübung bei Estern durch die Anwesenheit von Alkoholen ist verständlicherweise aus mehreren Gründen sehr unerwünscht. Sie kann beispielsweise die chemischen und physikalischen Eigenschaften des Esters wesentlich beeinträchtigen. Sie beeinträchtigt ferner das optische Aussehen. Dies alles führt zu einer merklichen Qualitätsminderung.

Die Alkoholreste in Estern können auch in gelöster Form vorliegen. Wenn in diesem Falle auch keine Trübung auftritt, sondern ein klarer Ester vorliegt, ist die Anwesenheit von Alkohol oft schon deswegen unerwünscht, weil dadurch die chemischen und physikalischen Eigenschaften des Esters verändert werden.

Aus der deutschen Patentschrift 1 181 692 und aus der deutschen Offenlegungsschrift 2423278 ist es bekannt, Alkoholreste aus einem Rohester durch eine unter bestimmten Bedingungen durchgeführte Wasserdampf-Destillation bzw. durch Extraktion mit Wasser zu entfernen. Diese Methoden sind nicht nur technisch aufwendig, mit ihnen wird auch eine zufriedenstellende Reinigung nicht immer erreicht. Aus der deutschen Patentschrift 1007314, der genannten deutschen Offenlegungsschrift 2423278 und der US-Patentschrift 3637774 ist es ferner bekannt, Ester zur Verbesserung der chemischen und physikalischen Eigenschaften mit Aktivkohle, Aluminiumoxid, Bleicherden und Kieselsäuregelen zu behandeln. Bezüglich der im Ester vorhandenen Alkohole bringen diese Mittel keine zufriedenstellende Reinigungswirkung.

In der englischen Patentschrift 705599 ist ein Verfahren zum Reinigen von Carbonsäureestern beschrieben, wobei der Rohester mit Methylendimethylether als Lösungsmittel versetzt und mit wässerigem Alkali und anschliessend mit Wasser gewaschen wird, worauf das Lösungsmittel abdestilliert wird. Es liegt auf der Hand, dass dieses Verfahren relativ umständlich ist. Ein anderes Ester-Reinigungsverfahren ist aus der deutschen Offenlegungsschrift 2326225 bekannt, in der ein Verfahren zur Reinigung organischer Ester von chemisch wirksamen und elektrisch leitenden Verunreinigungen wie Wasser, Alkohol und Säure beschrieben ist. Dabei wird der zu reinigende Ester mit anorganischen Materialien wie Molekularsieben, Fuller-Erde und Attapulgit-Ton behandelt, nachdem diese vorher in spezieller Weise aktiviert worden sind. Dieses Verfahren stellt zwar gegenüber dem oben erwähnten eine Verbesserung dar, ein Nachteil liegt jedoch bereits darin, dass es den erwähnten Aktivierungsschritt erfordert.

Aufgabe der Erfindung ist es daher, ein Verfahren anzugeben, mit dem eine Reinigung von Estern von Alkoholanteilen erreicht wird und das einfach durchführbar ist. Es sollen insbesondere die durch die Anwesenheit von Alkohol trüb erscheinenden Ester in klare Produkte übergeführt werden. Darüber hinaus soll es möglich sein, auch die im Ester gelösten Alkoholanteile weitgehend oder ganz zu entfernen.

Diese Aufgabe wird erfindungsgemäss dadurch gelöst, dass man die (alkoholhaltigen) Ester in flüssiger Form mit einer wirksamen Menge von wasserlöslichen Polysaccharidderivaten in fester Form behandelt.

Durch die erfindungsgemässe Behandlung wird überraschenderweise nicht nur der im Ester dispergierte und daher eine unerwünschte Trübung hervorrufende Alkohol schnell und in einfacher Weise entfernt, wodurch Ester von hoher Klarheit erhalten werden, sondern es kann auch der in gelöster Form vorliegende Alkohol, je nach Wunsch, weitgehend oder vollständig entfernt werden. Dieses Ergebnis war nicht zu erwarten, nachdem sich mit den verschiedensten bekannten Absorptionsmitteln, wie Aluminiumoxiden, Aktivkohlen, Kieselsäuregelen und Bleicherden, nicht einmal der dispergierte und schon gar nicht der gelöste Alkohol ohne weiteres entfernen lässt.

Bei der erfindungsgemässen Behandlung werden wasserlösliche Polysaccharidderivate in fester Form verwendet. Von diesen Polysaccharidderivaten werden vorzugsweise die Alkyl-, Hydroxyalkyl- und Carboxyalkylether der Cellulose, der Stärke und des Guar (Guar-Gum) sowie deren Mischether eingesetzt, wobei Alkyl eine Gruppe von vorzugsweise 1 bis 3 C-Atomen, Hydroxyalkyl eine Gruppe mit vorzugsweise 2 bis 4 C-Atomen und Carboxyalkyl eine Gruppe mit vorzugsweise 2 bis 3 C-Atomen ist (die Alkylgruppe in «Hydroxyalkyl» ist also vorzugsweise die Ethylen-, Propylen- oder Butylengruppe und die Alkylgruppe in «Carboxyalkyl» ist vorzugsweise $-CH_2-$ oder $-CH_2CH_2-$).

Die besonders bevorzugten Carboxyalkylether und Carboxyalkylmischether der Cellulose, des Guar und der Stärke können als solche oder in Form von Salzen, vorzugsweise von Alkalisalzen, insbesondere als Natriumsalze, eingesetzt werden, sie werden vorzugsweise in Salzform eingesetzt.

Als geeignete und zweckmässig anzuwendende Polysaccharidether seien einzeln genannt:
Methylcellulose, Ethylcellulose, Propylcellulose, Hydroxyethylcellulose, Hydroxypropylcellulose, Hydroxybutylcellulose, Hydroxyethylmethyl-cellulose, Hydroxy-ethyl-ethyl-cellulose, Hydroxypropyl-methyl-cellulose, Hydroxybutyl-methyl-cellulose, Carboxymethylcellulose, Carboxymethyl-methyl-cellulose, Hydroxyethyl-carboxy-methyl-cellulose und Hydroxy-propyl-carboxymethyl-cellulose und deren Natriumsalze sowie die analogen Guar- und Stärkeether.

Die erfindungsgemäss zu verwendenden Polysaccharidderivate sind bekannt und im Handel erhältlich. Sie liegen in der Regel in fester Form, und zwar als Pulver oder als Granulat vor, und sind, vorzugsweise bei Raumtemperatur, wasserlöslich. Ihre Viskosität, gemessen in einer 2%igen wässerigen Lösung, variiert innerhalb weiter Grenzen (vgl. Ullmanns Enzyklopädie der technischen Chemie, Band 9, 4. Auflage, Verlag Chemie, Weinheim, Bundesrepublik Deutschland).

Bei der erfindungsgemässen Behandlung werden wasserlösliche Polysaccharidderivate in fester Form, vorzugsweise als Pulver und/oder als Granulat, eingesetzt. Aus Zweckmässigkeitsgründen können die wasserlöslichen Polysaccharidderivate auch als Paste eingesetzt werden, die durch Anteigen mit wenig Wasser erhalten wird. Die Viskosität, der Grad der Substitution und die Substitutionsart der Polysaccharidderivate ist nicht kritisch, sofern sie wasserlöslich sind.

Die beim erfindungsgemässen Verfahren zu verwendende Menge an Polysaccharidderivat kann in weiten Grenzen variieren. Je nach gewünschtem Reinigungsgrad wird man eine jeweils wirksame Menge einsetzen. Wenn nur jener Alkoholanteil entfernt werden soll, auf den die Trübung des Esters zurückgeht, ist im allgemeinen eine relativ geringe Menge an Polysaccharidderivat erforderlich. Wenn hingegen neben dem die Trübung veranlassenden Alkoholanteil auch noch der gelöste Alkoholanteil entfernt werden soll, sind höhere Mengen erforderlich. Dies gilt auch dann, wenn der Alkohol nur in gelöster Form vorliegt und entfernt werden soll.

Die wirksame Menge an Polysaccharidderivat liegt demnach im allgemeinen bei 0,01 bis 20 Gew.-% vorzugsweise bei 0,1 bis 10 Gew.-%, bezogen auf das Gewicht des zu reinigenden Esters. Für die Entfernung des Trübungsalkohols reichen in der Regel 0,01 bis 5 Gew.-%, vorzugsweise 0,1 bis 3 Gew.-%, bezogen auf das Gewicht des zu reinigenden Esters. Für die Entfernung von Trübungsalkohol und gelöstem Alkohol oder von nur gelöstem Alkohol (falls Trübung nicht vorliegt) beträgt die Menge an Polysaccharidderivat im allgemeinen 5 bis 20 Gew.-%, vorzugsweise 10 bis 15 Gew.-%, bezogen auf das Gewicht des zu reinigenden Esters.

Beim erfindungsgemässen Verfahren werden die zu reinigenden Ester mit einer wirksamen Menge von wasserlöslichen, festen Polysaccharidderivaten behandelt. Dabei müssen die Ester in flüssiger Form vorliegen. Sofern sie nicht schon an sich flüssig sind, kann der flüssige Zustand einfach durch Erhitzen bis über den Schmelzpunkt des in fester oder nahezu fester (viskoser bis hochviskoser) Form vorliegenden Esters erreicht werden. Die erfindungsgemässe Behandlung kann nach verschiedenen Arbeitsweisen durchgeführt werden. Es kommt lediglich darauf an, dass der zu reinigende Ester in flüssiger Form mit dem zu verwendenden wasserlöslichen und in fester Form vorliegenden Polysaccharidderivat in Kontakt gebracht wird (ein- oder mehrmals, kontinuierlich oder diskontinuierlich), bis der gewünschte Reinigungsgrad erreicht ist, worauf das Polysaccharidderivat vorzugsweise durch Filtration oder durch Dekantierung abgetrennt wird, und der von Trübungsalkohol ganz und von gelöstem Alkohol wunschgemäss gereinigte (klare) Ester vorliegt. Das Inkontaktbringen wird in vorteilhafter Weise durch Rühren gefördert. Deshalb soll der Ester in flüssiger, d.h. gut fliessender und relativ leicht rührbarer Form vorliegen.

Die Zeit, die der zu reinigende Ester mit dem eingesetzten Polysaccharidderivat in Berührung ist, kann in weiten Grenzen variieren. Sie ist klarerweise von der Menge an eingesetztem Polysaccharidderivat und vom Vorliegen oder Nichtvorliegen einer Rührung abhängig und kann von wenigen Minuten bis zu mehreren Stunden reichen. Sie liegt in der Regel bei 5 bis 120 min, vorzugsweise bei 10 bis 60 min.

Die Temperatur während der Behandlung kann in weiten Grenzen variieren. Sie richtet sich im wesentlichen nach der Konstitution des zu reinigenden Esters. Ist dieser an sich flüssig, wird die Behandlung im allgemeinen bei Raumtemperatur (15 bis 25°C) durchgeführt.

Ist er fest oder sehr viskos, so wird die Behandlung bei jener Temperatur durchgeführt, bei der der Ester in flüssiger (niedrigviskoser, filtrierbarer) Form vorliegt, also bei einer entsprechend zweckmässigen Temperatur oberhalb des Schmelzpunktes des Esters. Die Temperatur des Esters bei der Behandlung liegt demnach zweckmässigerweise bei 15 bis 90°C. Aus Zweckmässigkeitsgründen wird die Behandlung im allgemeinen drucklos durchgeführt, sie kann aber auch unter Druck vorgenommen werden.

Die erfindungsgemässe Behandlung wird vorzugsweise in der Weise durchgeführt, dass dem flüssigen Ester in einem Gefäss unter Rühren auf einmal oder in Portionen pulverförmiges oder granulatförmiges Polysaccharidderivat zugesetzt wird. Nach dem Zusetzen wird in der Regel 5 bis 120 min, vorzugsweise 10 bis 60 min, weitergerührt. Anschliessend wird das Polysaccharidderivat abfiltriert oder seine Absetzung abgewartet und abdekantiert (wenn der Ester bei Raumtemperatur fest ist, kann nach dem Filtrieren oder Abdekantieren wieder abgekühlt werden).

Der nach dem erfindungsgemässen Verfahren behandelte Ester liegt – von Alkoholanteilen ganz oder teilweise gereinigt – als klare Flüssigkeit oder als Festprodukt vor, das im geschmolzenen Zustand ebenfalls eine klare Flüssigkeit darstellt.

Das erfindungsgemässe Verfahren ist auf beliebige Ester anwendbar. Es ist, wie oben bereits erwähnt, lediglich notwendig, dass sie während der erfindungsgemässen Behandlung in flüssiger Form vorliegen. Es wird vorzugsweise bei solchen Estern angewandt, deren Alkoholkomponente ein Alkohol ist, der mindestens eine Wasserstoffbrücken bildende Hydroxylgruppe besitzt und in der Säurekomponente im wesentlichen unlöslich ist; die in der Säurekomponente vorliegende Säure ist nicht kritisch.

Bei der Säurekomponente kann es sich um native oder synthetische Säure handeln. Sie ist in der Regel eine aliphatische Carbonsäure, vorzugsweise eine aliphatische Monocarbonsäure, die geradkettig oder verzweigt und gesättigt oder ungesättigt (vorzugsweise ein- bis dreifach ungesättigt) sein kann. Besonders bevorzugte Säurekomponenten sind Fettsäuren mit 4 bis 22 C-Atomen, vorzugsweise mit 8 bis 18 C-Atomen, wie Laurinsäure, Myristinsäure, Pamitinsäure, Stearinsäure, Ölsäure, Linolsäure und die entsprechenden Isosäuren.

Die Alkoholkomponente ist in der Regel ein aliphatischer Alkohol mit einer oder mehreren, vorzugsweise mit 1 bis 20 Hydroxylgruppen (einwertige und mehrwertige Alkohole), wobei die chemische Struktur der Monohydroxyalkohole und der Polyole (Diole, Triole usw.) an sich nicht kritisch ist. Sie ist vorzugsweise ein Alkohol, ausgewählt aus der Gruppe bestehend aus

a) Alkanolen mit 1 bis 4 C-Atomen und deren Oxyethylaten mit vorzugsweise 1 bis 20 Ethylenoxid-Einheiten pro Molekül;

b) Alkylenglykolen mit 2 bis 4 C-Atomen in der Alkylengruppe, deren Monoalkylethern mit 1 bis 4 C-Atomen in der Alkylgruppe sowie deren Oxethylaten mit vorzugsweise 1 bis 20 Ethylenoxid-Einheiten pro Molekül;

c) Glycerin und dessen Oxethylaten mit vorzugsweise 1 bis 20 Ethylenoxid-Einheiten pro Molekül;

d) Polyglycerinen, vorzugsweise Di- bis Dekaglycerin, und deren Oxethylaten mit vorzugsweise 1 bis 20 Ethylenoxid-Einheiten pro Molekül;

e) Erythrit und Pentaerythrit und deren Oxethylaten mit vorzugsweise 1 bis 20 Ethylenoxid-Einheiten pro Molekül;

f) Pentite und Hexite, vorzugsweise Mannit, Dulcit, Sorbit, Isosorbit und Sorbitan, und deren Oxethylaten mit vorzugsweise 1 bis 20 Ethylenoxid-Einheiten pro Molekül; und

g) Mono- und Disaccharide, vorzugsweise Fructose, Glucose, Lactose, Saccharose, und deren Oxethylaten mit vorzugsweise 1 bis 20 Ethylenoxid-Einheiten pro Molekül.

Besonders bevorzugte Alkoholkomponenten sind Glycerin, Di- bis Dekaglycerin, Sorbit und Sorbitan und deren Oxethylate mit 1 bis 20 Ethylenoxid-Einheiten pro Molekül sowie Oxethylate von Glucose, Lactose und Saccharose mit 1 bis 20 Ethylenoxid-Einheiten pro Molekül.

Das Verfahren, nach dem die Ester hergestellt worden sind, ist für die erfindungsgemässe Behandlung nicht kritisch. Sie können mit Veresterungs- und Umesterungsverfahren oder auch durch die Umsetzung von Säureanhydriden und Alkoholen, Säurehalogeniden und Alkoholen oder Nitrilen und Alkoholen erhalten worden sein (vgl. Ullmanns Enzyklopädie der technischen Chemie, Band 11, 4. Auflage, Verlag Chemie, Weinheim, Bundesrepublik Deutschland; deutsche Offenlegungsschrift 2423278 und US-Patentschrift 3637774). Die erfindungsgemäss zu behandelnden Ester sind vorzugsweise nach den bekannten und üblichen Veresterungs- und Umesterungsverfahren hergestellt worden. Bei der Herstellung der Ester können auch Mischungen der jeweiligen Ausgangssubstanzen, beispielsweise ein Carbonsäure- und ein Alkoholgemisch, eingesetzt worden sein. Für die erfindungsgemässe Behandlung ist es gleichgültig, ob Ester oder Mischester vorliegen und ob die Ester oder Mischester keine oder noch eine oder mehrere freie Hydroxylgruppen besitzen. Es kann sich auch um, beispielsweise durch Oxethylierung, modifizierte Ester handeln.

Das erfindungsgemässe Verfahren weist mehrere Vorteile auf. Es ist einfach und billig, bringt die gewünschte Reinigungswirkung und ist darüber hinaus von der Art, der Konstitution und von den physikalischen und chemischen Eigenschaften des Esters weitgehend unabhängig. Besonders vorteilhaft wird es angewandt, um solche Ester zu reinigen, die durch Destillation, Extraktion und/oder andere Trennverfahren bereits vorgereinigt worden sind, so dass sie nur noch relativ geringe Mengen an Alkohol enthalten, oder solche Ester, die aus einer unter stöchiometrischen Mengenverhältnissen durchgeführten Umsetzung von Alkohol und Säure stammen und daher von Haus aus wenig Alkohol enthalten.

Die Erfindung wird nun an Beispielen noch näher erläutert:

*Beispiel 1:*

Durch die bekannte und übliche Umsetzung von 1 mol Diglycerin mit 2 mol Isostearinsäure wurde zunächst ein Rohester hergestellt. Der erhaltene, bei Raumtemperatur flüssige Rohester war stark trüb.

500 g dieses Rohesters wurden in einem mit Rührer versehenen 1-Liter-Becherglas vorgelegt und bei Raumtemperatur unter Rühren mit 0,5 g pulverförmiger Methylhydroxyethylcellulose (das sind 0,1 Gew.-%, bezogen auf Ester) versetzt. Anschliessend wurde noch 15 min bei Raumtemperatur weitergerührt und dann abfiltriert. Der ursprünglich trübe Ester stellte nun eine klare Flüssigkeit dar.

*Beispiel 2:*

Es wurde zunächst nach bekannter und üblicher Arbeitsweise durch Reaktion von 1 mol Diglycerin mit 2 mol Stearinsäure und anschliessende Oxethylierung mit 3 mol Ethylenoxid ein Diglycerinstearinsäureester-oxethylat hergestellt. Der erhaltene, bei Raumtemperatur wachsartige Rohester hatte einen Schmelzpunkt von 50 bis 60°C und war im geschmolzenen Zustand eine stark trübe Flüssigkeit.

1 kg dieses Rohesters wurde in einem 2-Liter-Becherglas auf 75°C erhitzt. Hierauf wurden unter Rühren und Halten auf 75°C 5 g granuliertes Carboxymethylhydroxypropylguar (das sind 0,5 Gew.-%, bezogen auf Esteroxethylat) zugesetzt und 30 min bei 75°C weitergerührt. Anschliessend wurde abfiltriert. Der ursprünglich stark trübe Ester war nun im geschmolzenen Zustand eine ganz klare Flüssigkeit.

*Beispiel 3:*

Ein rohes Glycerin-monostearat, das durch Glycerinolyse von Glycerintristearat nach üblichen Methoden erhalten worden war, enthielt nach dem Abtrennen der Hauptmenge an überschüssigem Glycerin noch 9 Gew.-% Restglycerin, das im Monostearat zum Grossteil in gelöster Form vorlag.

500 g Rohprodukt wurden in einem 2-Liter-Becherglas auf 75°C erhitzt und damit in eine dünnflüssige Form gebracht. Unter Rühren wurden dann bei 75°C 50 g pulverförmige Carboxymethylcellulose (das sind 10 Gew.-%, bezogen auf den Rohester) portionsweise zugesetzt, worauf bei 80°C 45 min lang weitergerührt wurde. Anschliessend wurde, um die Dünnflüssigkeit und damit die Filtrierbarkeit aufrechtzuerhalten, bei einer Temperatur von 75 bis 80°C filtriert. Der ursprünglich trübe Ester war nun im geschmolzenen (flüssigen) Zustand ein völlig klares Produkt, das nur noch 2,7 Gew.-% Restglycerin gelöst enthielt. Dieses Beispiel wurde unter Einsatz der Carboxymethylcellulose als Paste wiederholt, die durch Anteigen von 15 g pulverförmiger Carboxymethylcellulose mit 25 ml Wasser erhalten worden war. Der Restglyceringehalt betrug nur noch 3,2 Gew.-%.

*Beispiel 4:*

Durch die bekannte und übliche Umsetzung von 1 mol Pentaerythrit mit 2 mol Ölsäure wurde zunächst ein Rohester hergestellt. Er enthielt noch etwa 5 Gew.-% festes, nicht umgesetztes Pentaerythrit und stellte eine bei Raumtemperatur relativ hochviskose und trübe Flüssigkeit dar. Wegen des sehr viskosen Zustandes ist die Abtrennung des Pentaerythritanteils bei Raumtemperatur durch Filtration nicht möglich. Beim Erwärmen auf etwa 80°C wird das Produkt zwar flüssiger und damit filtrierbar, dabei geht jedoch das bei Raumtemperatur ungelöst vorliegende Pentaerythrit in Lösung, wodurch seine Abtrennung auch durch Filtration in der Wärme nicht erreicht werden kann. Mit dem erfindungsgemässen Verfahren ist es aber möglich, den vorliegenden Rohester schnell und in einfacher Weise von den Pentaerythritanteilen zu reinigen und einen klaren Ester zu erhalten.

Zu 500 g Rohester wurden in einem 1-Liter-Becherglas bei 80°C unter Rühren 35 g pulverförmige Carboxymethylstärke (das sind 7 Gew.-%, bezogen auf den Rohester) zugesetzt und 45 min lang weitergerührt. Anschliessend wurde heiss (das heisst bei 80°C) filtriert. Der so erhaltene Ester war nun bei Raumtemperatur eine hochviskose klare Flüssigkeit.

*Beispiel 5:*

Es wurde zunächst ein Rohester hergestellt, indem nach bekannter und üblicher Arbeitsweise Sorbit mit 15 mol Ethylenoxid oxethyliert und anschliessend mit 3 mol Laurinsäure verestert wurde. Der Rohester war bei Raumtemperatur dickflüssig und stark trüb.

500 g des Rohesters wurden in einem mit einer Stickstoffeinleitungsvorrichtung und einem Rührer ausgestatteten 1-Liter-Kolben vorgelegt und auf 80°C erhitzt. Nun wurden unter Rühren 10 g pulverförmige Hydroxyethylstärke (das sind 2 Gew.-%, bezogen auf Rohester) langsam zugegeben und hierauf 90 min bei 80°C weitergerührt. Anschliessend wurde bei 80°C abfiltriert. Es wurde eine bei Raumtemperatur hochviskose, ganz klare Flüssigkeit erhalten.

*Beispiel 6:*

Eine nach üblichen Methoden mit 9 mol Ethylenoxid oxethylierte und anschliessend mit 1 mol Isononansäure nach bekannten Verfahren veresterte Saccharose (Rohester) wurde wie folgt gereinigt:

200 g des Rohesters wurden in einem 500-ml-Kolben (versehen mit Stickstoffeinleitungsvorrichtung und Rührer) vorgelegt. Der Rohester wurde auf 75°C erhitzt, unter Rühren mit 2 g granuliertem Carboxymethylhydroxypropylguar (das ist 1 Gew.-%, bezogen auf Rohester) versetzt und 30 min bei 75°C weitergerührt. Anschliessend wurde heiss (bei 75°C) filtriert. Das bei Raumtemperatur viskose Produkt war klar.

**Patentansprüche**

1. Verfahren zum Reinigen von alkoholhaltigen Estern, dadurch gekennzeichnet, dass man die Ester in flüssiger Form mit einer wirksamen Menge von wasserlöslichen Polysaccharidderivaten in fester Form behandelt.

2. Verfahren nach Anspruch 1, dadurch gekennzeichnet, dass man wasserlösliche und pulver- oder granulatförmige Polysaccharidderivate, ausgewählt aus der Gruppe bestehend aus Alkylethern, Hydroxyalkylethern, Carboxyalkylethern und Alkyl-, Hydroxyalkyl- und Carboxyalkyl-Mischethern der Cellulose, der Stärke und des Guar, einsetzt.

3. Verfahren nach Anspruch 2, dadurch gekennzeichnet, dass man Ether einsetzt, in denen die Alkylgruppe 1 bis 3 C-Atome, die Hydroxyalkylgruppe 2 bis 4 C-Atome und die Carboxyalkylgruppe 2 bis 3 C-Atome besitzt.

4. Verfahren nach Anspruch 3, dadurch gekennzeichnet, dass man Carboxyalkylether oder Carboxyalkyl-Mischether der Cellulose, der Stärke und des Guar oder deren Alkalisalze einsetzt.

5. Verfahren nach Anspruch 1, dadurch gekennzeichnet, dass man die Polysaccharidderivate in einer Menge von 0,01 bis 20 Gew.-%, bezogen auf das Gewicht des alkoholhaltigen Esters, einsetzt.

6. Verfahren nach Anspruch 1, dadurch gekennzeichnet, dass die Behandlung in der Weise durchgeführt wird, dass man den flüssigen Ester mit pulverförmigen oder granulatförmigen Polysaccharidderivat unter Rühren in Kontakt bringt, bis der gewünschte Reinigungsgrad erreicht ist, und anschliessend das Polysaccharidderivat abfiltriert oder abdekantiert.

7. Verfahren nach Anspruch 1, dadurch gekennzeichnet, dass man als Ester solche einsetzt, bei denen die Säurekomponente eine geradkettige oder verzweigte und gesättigte oder ein- bis dreifach ungesättigte aliphatische Monocarbonsäure und die Alkoholkomponente ein aliphatischer, ein- oder mehrwertiger Alkohol ist.

## Claims

1. A process for the purification of esters containing alcohols, which comprises treating the esters in the liquid form with an effective amount of water-soluble polysaccharide derivatives in the solid form.

2. The process as claimed in claim 1, wherein water-soluble polysaccharide derivatives in the form of a powder or granules, selected from the group consisting of alkyl ethers, hydroxyalkyl ethers, carboxyalkyl ethers and mixed alkyl, hydroxyalkyl and carboxyalkyl ethers of cellulose, of starch and of guar, are used.

3. The process as claimed in claim 2, wherein ethers are used in which the alkyl group has 1 to 3 C atoms, the hydroxyalkyl group has 2 to 4 C atoms and the carboxyalkyl group has 2 to 3 C atoms.

4. The process as claimed in claim 3, wherein carboxyalkyl ethers or mixed carboxyalkyl ethers of cellulose of starch and of guar, or their alkali metal salts are used.

5. The process as claimed in claim 1, wherein the polysaccharide derivatives are used in an amount of 0.01 to 20% by weight, relative to the weight of the ester, containing alcohol.

6. The process as claimed in claim 1, wherein the treatments is carried out in such a manner that the liquid ester is brought into contact with a polysaccharide derivative in the form of a powder or granules, with stirring, until the desired degree of purity is achieved, and then the polysaccharide derivative is filtered off or separated by decantation.

7. The process as claimed in claim 1, wherein the esters used are those in which the acid component is a straight-chained or branched, saturated or singly to triply unsaturated, aliphatic monocarboxylic acid and the alcohol component is an aliphatic monohydric or polyhydric alcohol.

## Revendications

1. Procédé de purification d'esters contenant des alcools, procédé caractérisé en ce qu'on traite les esters à l'état liquide par une quantité efficace de dérivés de polysaccharides qui sont solubles dans l'eau et se trouvent à l'état solide.

2. Procédé selon la revendication 1, caractérisé en ce qu'on utilise des dérivés de polysaccharides hydrosolubles qui sont sous forme de poudres ou de granulés et qui sont choisis dans l'ensemble constitué par les éthers alkyliques, les éthers hydroxyalkyliques, les éthers carboxyalkyliques et les éthers mixtes alkyliques, hydroxyalkyliques et carboxyalkyliques de la cellulose, de l'amidon et du guar.

3. Procédé selon la revendication 2, caractérisé en ce qu'on utilise des éthers dans lesquels le radical alkyle contient de 1 à 3 atomes de carbone, le radical hydroxyalkyle contient de 2 à 4 atomes de carbone et le radical carboxyalkyle contient de 2 à 3 atomes de carbone.

4. Procédé selon la revendication 3, caractérisé en ce qu'on utilise des éthers carboxyalkyliques ou des éthers mixtes carboxyalkyliques de la cellulose, de l'amidon et du guar ou leurs sels de métaux alcalins.

5. Procédé selon la revendication 1, caractérisé en ce qu'on utilise les dérivés de polysaccharides en une quantité de 0,01 à 20% en poids par rapport au poids de l'ester contenant de l'alcool.

6. Procédé selon la revendication 1, caractérisé en ce qu'on effectue le traitement de la façon suivante: on met en contact l'ester liquide, tout en agitant, avec le dérivé de polysaccharide sous forme pulvérulente ou granulée, jusqu'à ce que le degré de purification souhaité soit atteint, puis on sépare le dérivé de polysaccharide par filtration ou par décantation.

7. Procédé selon la revendication 1, caractérisé en ce qu'on utilise des esters dans lesquels la composante acide est un acide monocarboxylique aliphatique linéaire ou ramifié qui est saturé ou qui contient de 1 à 3 insaturations, et la composante alcool est un monoalcool ou un polyol aliphatique.